(19) Europäisches Patentamt
European Patent Office
Office européen des brevets

(11) **EP 1 835 950 B1**

(12) **EUROPÄISCHE PATENTSCHRIFT**

(45) Veröffentlichungstag und Bekanntmachung des Hinweises auf die Patenterteilung:
**04.12.2013 Patentblatt 2013/49**

(21) Anmeldenummer: **05796447.0**

(22) Anmeldetag: **15.10.2005**

(51) Int Cl.:
***A61M 1/16*** *(2006.01)*   ***A61M 1/36*** *(2006.01)*

(86) Internationale Anmeldenummer:
**PCT/EP2005/011103**

(87) Internationale Veröffentlichungsnummer:
**WO 2006/072271 (13.07.2006 Gazette 2006/28)**

(54) **VORRICHTUNG UND VERFAHREN ZUR ERKENNUNG VON KOMPLIKATIONEN WÀHREND EINER EXTRAKORPORALEN BLUTBEHANDLUNG**

DEVICE AND METHOD FOR DETECTING COMPLICATIONS DURING AN EXTRACORPOREAL BLOOD TREATMENT

DISPOSITIF ET PROCEDE POUR IDENTIFIER DES COMPLICATIONS AU COURS D'UN TRAITEMENT SANGUIN EXTRACORPOREL

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **07.01.2005   DE 102005001051**

(43) Veröffentlichungstag der Anmeldung:
**26.09.2007   Patentblatt 2007/39**

(73) Patentinhaber: **Fresenius Medical Care Deutschland GmbH**
**61352 Bad Homburg v.d.H. (DE)**

(72) Erfinder:
• **DANIEL, Pia**
**78351 Bodman (DE)**
• **MÜLLER, Carsten**
**97502 Euerbach (DE)**

(74) Vertreter: **Oppermann, Frank et al**
**OANDO Oppermann & Oppermann LLP**
**John-F.-Kennedy-Straße 4**
**65189 Wiesbaden (DE)**

(56) Entgegenhaltungen:
**DE-A1- 19 747 360     DE-C1- 19 702 441**
**US-A- 5 588 959**

**Beschreibung**

Die Erfindung bezieht sich auf eine Vorrichtung zur Erkennung von Komplikationen während einer extrakorporalen Blutbehandlung mit einer Blutbehandlungsvorrichtung, bei der das zu behandelnde Blut in einem extrakorporalen Blutkreislauf die Blutkammer eines durch eine semipermeable Membran in die Blutkammer und eine Dialysierflüssigkeitskammer unterteilten Dialysators durchströmt und Dialysierflüssigkeit in einem Dialysierflüssigkeitskreislauf die Dialysierflüssigkeitskammer des Dialysators durchströmt. Darüber hinaus betrifft die Erfindung ein Verfahren zur Erkennung von Komplikationen während einer extrakorporalen Blutbehandlung.

Bei Verfahren der chronischen Blutreinigungstherapie, beispielsweise bei der Hämodialyse, Hämofiltration und Hämodiafiltration, wird Blut über einen extrakorporalen Blutkreislauf geleitet. Als Zugang zum Blutgefäßsystem wird häufig operativ eine arteriovenöse Fistel angelegt. Ebenso ist der Einsatz eines Implantats möglich.

Für die Funktionsfähigkeit der Fistel ist deren Perfusion von Bedeutung. Sinkt der Fistelfluss unter einen kritischen Wert, dann steigt das Risiko einer Fistelthrombose mit dem möglichen Verlust des Gefäßzuganges, was in der Dialysebehandlung eine erhebliche Komplikation darstellt. Ist der Fistelfluss während der Dialysebehandlung unzureichend und kleiner als der extrakorporale Blutfluss, kommt es zur lokalen Fistelrezirkulation, wobei eine Fraktion des dialysierten und mit der venösen Blutleitung zur Fistel zurückgeführten Blutes über die arterielle Blutleitung dem Dialysator wieder zugeführt wird. Die Fistelrezirkulation bewirkt eine bedeutende Verminderung der Dialyseeffizienz (F. Gotch: "Models to predict recirculation and its effect on treatment time in single-needle-dialysis", First Intl. Symposium on Single-Needle-Dialyse, Hrsg.: S. Rignoir. R. Vanholder and P. Invanovich, Cleveland, ISAO Press, 1984, Seite 305 ff. ). Daher wird die Messung der Qualität des Gefäßzuganges als ein wichtiges Mittel zur Qualitätssicherung bei der Dialysebehandlung angesehen.

Neben der Fistelrezirkulation zirkuliert ein kleiner Teil des Blutes bei derartigen Gefäßzugängen auch immer direkt über den Blutkreislauf des Patienten zum Gefäßzugang, ohne am Stoffwechsel in den Kapillarsystemen teilnehmen zu können. Diese Rezirkulationsart wird kardiopulmonale Rezirkulation genannt und beträgt überlicherweise wenige Prozent.

Aufgrund ihrer klinischen Bedeutung sind eine Reihe von Verfahren zur Messung der Rezirkulation bekannt. Die bekannten Verfahren beruhen im Allgemeinen auf einer Messung einer physikalischen oder chemischen Kenngröße des Blutes, die im venösen Zweig des extrakorporalen Kreislaufs verändert wird. Die physikalische oder chemische Kenngröße des Blutes kann unmittelbar durch einen manuellen Eingriff in den extrakorporalen Blutkreislauf oder auch mittelbar über einen Eingriff in den Dialysierflüssigkeitskreislauf verändert werden.

Aus dem Artikel von Krämer und Polaschegg in der Zeitschrift EDTNA-ERCA Journal Vol. XIX, Nr. 2, S. 8 - 15, April 1993 ist ein als Thermodilution bezeichnetes Verfahren zur Rezirkulationsmessung bekannt. Bei dem bekannten Verfahren wird ein kurzzeitiger Temperaturabfall im Diaylsierflüssigkeitskreislauf initiiert, der sich auf den venösen Zweig des extrakorporalen Blutkreislaufs überträgt und zu einem nachweisbaren Temperatursprung im arteriellen Zweig des extrakorporalen Kreislaufs dann führt, wenn eine Rezirkulation auftritt.

Eine bekannte Vorrichtung zur Durchführung des als Thermodilution bezeichneten Verfahrens weist einen im arteriellen Zweig und venösen Zweig des extrakorporalen Kreislaufs angeordneten Temperaturfühler auf. Mit dem venösen Temperaturfühler wird der Temperatursprung aufgenommen, der auf den im Dialysierflüssigkeitskreislauf erzeugten Temperaturabfall zurückzuführen ist. Der gemessene Temperatursprung wird zeitlich integriert und nachfolgend mit dem im arteriellen Messfühler registrierten Temperaturverlauf verglichen. Das Verhältnis der beiden Temperatur-Integrale zueinander ist ein Maß für die gesamte Effizienzminderung der Dialysebehandlung durch fistel- und kardiopulmonäre Rezirkulation.

Die bekannte Vorrichtung zur Rezirkulationsmessung hat sich in der Praxis bewährt. Ein entscheidender Nachteil liegt jedoch darin, dass die Rezirkulation auf der Grundlage einer gemessenen Veränderung einer physikalischen oder chemischen Kenngröße des Blutes im Blutkreislauf bestimmt wird. Dies ist insofern nachteilig, als im Blutkreislauf Temperaturfühler anzuordnen sind. Dadurch ergibt sich ein verhältnismäßig hoher apparativer Aufwand. Des weiteren erfordert die Messung mit dem Temperaturbolus relativ viel Zeit. Daher wird die Rezirkulationsmessung mit Temperaturbolus auch nicht kontinuierlich während der Behandlung, sondern oft nur auf manuelle Anforderung hin durchgeführt.

Die DE 197 02 441 C1 zeigt die Nachteile des als Thermodilution bezeichneten Verfahrens auf. Zur Verringerung des apparativen Aufwandes des extrakorporalen Blutbehandlungsvorrichtung schlägt die DE 197 02 441 ein Verfahren zur Bestimmung der Rezirkulation auf der Grundlage der Veränderung einer physikalischen oder chemischen Kenngröße in der Dialysierflüssigkeit stromauf des Dialysators und der Erfassung der Änderung einer physikalischen oder chemischen Kenngröße in der Dialysierflüssigkeit stromab des Dialysators vor.

Neben der Rezirkulationsmessung sind verschiedene Verfahren zur Messung der Dialysance oder Clearance bekannt, die Anhaltspunkte für die Effizienz einer Dialysebehandlung geben. Die US 6,702,774 B1 beschreibt ein Verfahren zur Messung der Dialysance oder Clearance während einer extrakorporalen Blutbehandlung. Im Gegensatz zu dem als Thermodilution bezeichneten Verfahren zur Rezirkulationsmessung erfolgt die Bestimmung der Dialysance oder Clearance auf der Grundlage einer gemessenen physikalischen oder chemischen Kenngröße der Dialysierflüssigkeit im Dialysierflüssigkeitskreislauf. Folglich ist eine Messung am extrakorporalen Blutkreislauf nicht erforderlich. Bei dem bekannten Verfahren wird stromauf des Dialysators eine physikalische oder chemische Kenngröße der Dialysierflüssigkeit in einem vorgegebenen Zeitintervall verändert und die Änderung der physikalischen oder chemischen Kenngröße stromab des Dialysators gemessen. Vorzugsweise ist die physikalische oder chemische Kenngröße die Dialysierflüssigkeits-Ionenkonzentration, die mit einer Leitfähigkeitsmessung der Dialysierflüssigkeit bestimmt wird. Die Dialysance wird aus dem Integral über die Zeit der gemessenen Dialysierflüssigkeits-Ionenkonzentration stromauf des Dialysators und dem Integral über die Zeit der gemessenen Ionenkonzentration stromab des Dialysators allein aus den dialysierflüssigkeitsseitigen Größen bestimmt.

Die DE 197 47 360 A1 beschreibt ein Verfahren zur Bestimmung der Dialysance und Clearance, bei dem stromauf des Dialysators der Dialysierflüssigkeit als Bonus eine vorbestimmte Menge einer Substanz zugegeben wird, deren Dialysance bestimmt werden soll. Die im Dialysator nicht dialysierte Stoffmenge wird durch Integration der mit einem Sensor stromab des Dialysators gemessenen Stoffkonzentration über die Zeit bestimmt und aus zugegebener Stoffmenge, stromab nachgewiesener Stoffmenge sowie dem Dialysierflüssigkeitsfluß die Dialysance ermittelt. Die DE 197 47 360 A1 führt als Stand der Technik das aus der DE 39 38 662 beschriebene Verfahren an, mit dem nur die effektive Clearance, nicht aber die Dialysator Clearance gemessen werden kann, wobei sich beide Werte durch den Einfluss der Rezirkulation unterscheiden sollen.

Der Erfindung liegt die Aufgabe zugrunde, eine Vorrichtung bereitzustellen, mit der während einer extrakorporalen Blutbehandlung Komplikationen mit hoher Sicherheit erkannt werden können. Die Lösung dieser Aufgabe erfolgt mit den Merkmalen des Patentanspruchs 1.

Darüber hinaus ist eine Aufgabe der Erfindung, ein Verfahren anzugeben, das die Erkennung von Komplikationen mit hoher Sicherzeit während einer extrakorporalen Blutbehandlung ermöglicht. Diese Aufgabe wird mit den Merkmalen des Patentanspruchs 11 gelöst.

Vorteilhafte Ausgestaltungen der Erfindung sind Gegenstand der Unteransprüche.

Die erfindungsgemäße Vorrichtung und das erfindungsgemäße Verfahren beruhen auf der Kombination der Überwachung der Dialysance oder Clearance auf der Grundlage von dialysatseitigen Messgrößen und der Überwachung der Rezirkulation auf der Grundlage von blutseitigen Messgrößen. Während der Blutbehandlung wird zu aufeinanderfolgenden Zeitpunkten die Dialysance oder Clearance auf der Grundlage von dialysatseitigen Messungen überwacht, ohne dass ein Eingriff in den extrakorporalen Blutkreislauf erforderlich ist. Die Überwachung der Rezirkulation auf der Grundlage von blutseitigen Messungen erfolgt erst dann, wenn eine Veränderung der Dialysance oder Clearance festgestellt worden ist.

Die erfindungsgemäße Vorrichtung bzw. das Verfahren basieren auf der Erkenntnis, dass eine Rezirkulation mit einer Veränderung, insbesondere einer Verringerung der Dialysance oder Clearance verbunden ist. Andererseits kann eine Verringerung der Dialysance oder Clearance auch auf andere Komplikationen als die einer Rezirkulation zurückzuführen sein. Bei der erfindungsgemäßen Vorrichtung bzw. dem Verfahren wird daher nach der Feststellung einer Veränderung der Dialysance oder Clearance mit einer zweiten Messung überprüft, ob die Ursache der Veränderung tatsächlich in dem Auftreten einer Rezirkulation liegt.

Da die Rezirkulationsmessung auf der Grundlage der blutseitigen Größen nur dann erfolgt, wenn eine derartige Komplikation angenommen wird, kann die Überwachung auf der Grundlage der dialysierflüssigkeitsseitigen Größen kontinuierlich oder quasi kontinuierlich vorgenommen werden.

Die hohe Sicherheit des erfindungsgemäßen Verfahrens ist damit zu begründen, dass auf eine Rezirkulation erst dann geschlossen wird, wenn zwei voneinander unabhängige Messverfahren darauf hinweisen.

Die erfindungsgemäße Vorrichtung bzw. das Verfahren erlaubt die Unterscheidung zwischen Komplikationen, die auf eine Rezirkulation zurückzuführen ist, beispielsweise Komplikationen mit dem Gefäßzugang, oder Komplikationen, die andere zu einer Veränderung der Dialysance oder Clearance führende Ursachen haben, beispielsweise eine verringerte Effizienz des Dialysators.

Bei einer bevorzugten Ausführungsform verfügt die erfindungsgemäße Vorrichtung über eine Auswerteinrichtung, die beim Auftreten von Komplikationen ein Steuersignal für einen Alarm oder einen Eingriff in die Maschinensteuerung der Behandlungsvorrichtung erzeugt. Vorzugsweise erzeugt die Auswerteinrichtung ein erstes Steuersignal, das auf die Veränderung der Dialysance oder Clearance aufgrund einer Rezirkulation hinweist und ein zweites Steuersignal das auf eine Veränderung der Dialysance hinweist, die nicht die Folge einer Rezirkulation ist.

Eine besonders bevorzugte Ausführungsform sieht vor, dass die Auswerteinrichtung eine Speichereinheit zum Speichern eines vorgegebenen Wertes einer Dialysance, Clearance oder Rezirkulation und eine Vergleichseinheit zum Vergleichen des zu einem bestimmten Zeitpunkt ermittelten Dialysance-, Clearance- oder Rezirkulationswertes mit dem vorgegebenen Wert aufweist. Eine Veränderung der Rezirkulation wird dann festgestellt, wenn die Differenz zwischen dem vorgegebenen und zu einem bestimmten Zeitpunkt ermittelten Wert größer als ein vorgegebener Grenzwert ist. Der vorgegebene Wert kann ein absoluter Wert oder der bei einer vorausgehenden Messung ermittelte Wert sein.

Die Bestimmung der Rezirkulation auf der Grundlage der blutseitigen Größen erfolgt vorzugsweise mit dem als Thermodilution bezeichneten Verfahren, das aus der eingangs erwähnten Zeitschrift EDTNA-ERCA bekannt ist, auf die zum Zwecke der Offenbarung ausdrücklich Bezug genommen wird. Vorzugsweise erfolgt die Bestimmung der Dialysance oder Clearance nach dem aus der eingangs erwähnten US 6,702,774 B1 bekannten Verfahren, auf die ebenfalls ausdrücklich Bezug genommen wird.

Grundsätzlich können aber auch andere Verfahren angewandt werden, so lange die kontinuierliche oder quasi kontinuierliche Messung nur auf der Grundlage von dialysatseitigen Größen und die nur bei der Annahme einer Komplikation durchgeführte Messung auf der Grundlage von blutseitigen Größen erfolgt.

Die erfindungsgemäße Vorrichtung kann eine selbständige Einheit bilden, die für eine Blutbehandlungsvorrichtung zur Verfügung gestellt wird. Da aber die wesentlichen Komponenten der erfindungsgemäßen Vorrichtung als solche, zu denen insbesondere die schaltungstechnischen Komponenten für die Messung und Auswertung gehören, beispielsweise Temperatur- und Leitfähigkeitssensoren, Mikroprozessoren etc., bereits in den bekannten Blutbehandlungsvorrichtungen im Allgemeinen vorhanden sind, kann die erfindungsgemäße Vorrichtung auch ohne hohen zusätzlichen Aufwand in die Blutbehandlungsvorrichtung integriert werden.

Eine Blutbehandlungsvorrichtung mit einer derartigen Vorrichtung zur Erkennung von Komplikationen ist Gegenstand des Patentanspruchs 8.

Im Folgenden wird ein Ausführungsbeispiel der Erfindung unter Bezugnahme auf die Zeichnungen näher erläutert.

Es zeigen:

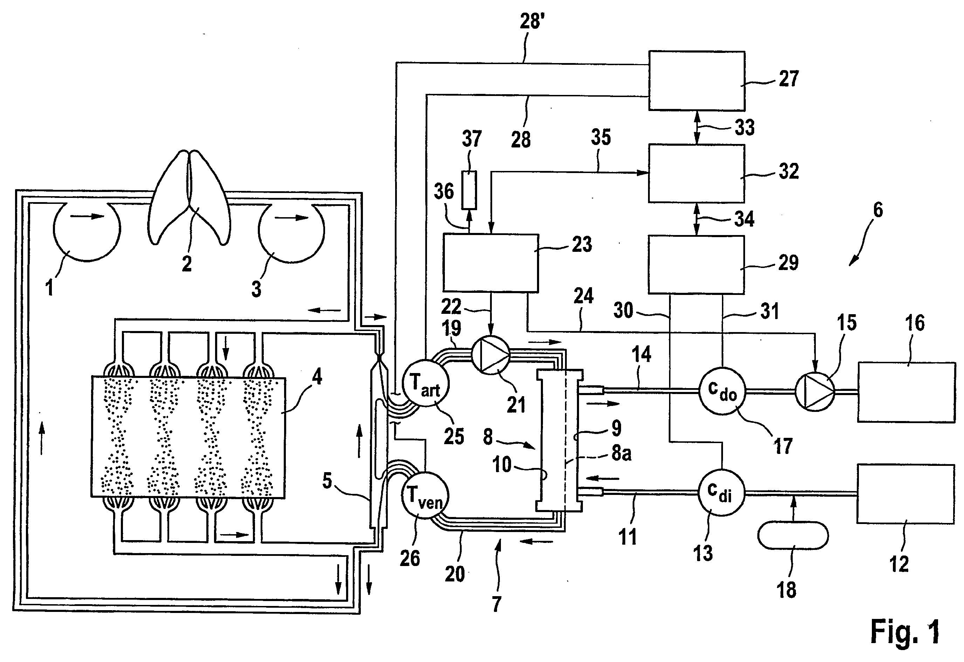

Figur 1    eine schematische Darstellung der wesentlichen Komponenten einer Vorrichtung zur extrakorporalen Blutbehandlung mit einer Vorrichtung zur Erkennung von Komplikationen während der Blutbehandlung,

Figur 2    den zeitlichen Verlauf der Temperatur des Blutes im arteriellen und venösen Zweig des extrakorporalen Blutkreislaufs nach einem dialysatseitigen Temperaturbolus und

Figur 3    den zeitlichen Verlauf der gemessenen Dialysierflüssigkeits-Ionenkonzentration stromauf und stromab des Dialysators nach einem Konzentrationsbolus.

Nachfolgend wird die erfindungsgemäße Vorrichtung zusammen mit den wesentlichen Komponenten einer Dialysevorrichtung einschließlich des intrakorporalen Kreislaufs beschrieben. Die erfindungsgemäße Vorrichtung kann aber auch eine separate Baugruppe bilden.

Der intrakorporale Kreislauf umfasst das rechte Ventrikel 1 des Herzens, die Lunge 2, das linke Ventrikel 3 und sämtliche Kapillarsysteme 4 des Körpers in inneren Organen, Muskulatur und Haut etc. Um einen Zugang zu dem Blutgefäßsystem zu schaffen, ist eine arterio-venöse Fistel 5 angelegt.

Die Dialysevorrichtung besteht im Wesentlichen aus einem Dialysierflüssigkeitskreislauf 6 und einem extrakorporalen Blutkreislauf 7, zwischen denen sich ein Dialysator 8 mit einer Dialysierflüssigkeitskammer 9 und einer Blutkammer 10 befindet, die durch eine semipermeable Membran 8a voneinander getrennt sind. Die Dialysierflüssigkeitskammer 9 ist stromauf des Dialysators 8 über eine Dialysierflüssigkeitszuführleitung 11 mit einer Dialysierflüssigkeitsquelle 12 verbunden. In die Dialysierflüssigkeitszuführleitung ist eine Messeinrichtung 13 mit einem Leitfähigkeitssensor oder einem optischen Sensor zur Bestimmung der Dialysierflüssigkeits-Eingangskonzentration $C_{di}$ geschaltet. Stromab des Dialysators 8 ist an die Dialysierflüssigkeitskammer 9 eine Dialysierflüssigkeitsabführleitung 14 angeschlossen, die eine Dialysierflüssigkeitspumpe 15 aufweist und die in einen Abfluss 16 führt. In die Dialysierflüssigkeitsabführleitung 14 ist eine Messeinrichtung 17 mit einem Leitfähigkeitssensor oder einem optischen Sensor zur Bestimmung der Dialysierflüssigkeits-Ausgangskonzentration $C_{do}$ geschaltet.

Mit dem Bezugszeichen 18 ist eine stromauf des Dialysators 8 angeordnete Einrichtung bezeichnet, mit der eine physikalische oder chemische Kenngröße der in den Dialysator fließenden Dialysierflüssigkeit verändert, insbesondere ein Bolus einer Substanz, d.h. ein Konzentratbolus aufgegeben werden kann.

Der extrakorporale Blutkreislauf 7 umfasst einen arteriellen Zweig 19, der mit dem arteriellen Teil der Fistel 5 in Verbindung steht, die Blutkammer 10 des Dialysators 8 und einen venösen Zweig 20, der mit dem venösen Teil der Fistel in Verbindung steht. Im arteriellen Zweig 19 ist eine Blutpumpe 21 angeordnet, die über eine Steuerleitung 22 mit einer Steuereinheit 23 verbunden ist, mit der die Förderrate der Blutpumpe 21 verändert werden kann. Die Steuereinheit 23 steht über eine weitere Steuerleitung 24 mit der Dialysierflüssigkeitspumpe 15 in Verbindung.

In den arteriellen Zweig 19 des extrakorporalen Blutkreislaufs 7 ist eine Messeinrichtung 25 zum Messen der arteriellen Bluttemperatur und in den venösen Zweig eine Messeinrichtung 26 zum Messen der venösen Bluttemperatur geschaltet.

Die erfindungsgemäße Vorrichtung verfügt über eine erste und zweite Einrichtung zur Überwachung der Rezirkulation. Die erste Überwachungseinrichtung 27 empfängt über Signalleitungen 28, 28' die Messwerte der mit der arteriellen und venösen Messeinrichtung 25, 26 gemessenen arteriellen und venösen Bluttemperatur. Die zweite Überwachungseinrichtung 29 empfängt über Signalleitungen 30, 31 die Messwerte $C_{di}$, $C_{do}$ der Messeinrichtungen 13, 17 für die Ionenkonzentration stromauf und stromab des Dialysators.

Eine Auswerteinrichtung 32 empfängt über Datenleitungen 33, 34 die in den Überwachungseinrichtungen 27, 29 ermittelten Daten. Die Auswerteinrichtung 32 steht mit einer weiteren Datenleitung 35 mit der Steuereinheit 23 in Verbindung. Über eine Signalleitung 36 ist an die Steuereinheit 23 eine akustische und/oder optische Anzeige- oder Alarmeinrichtung 37 angeschlossen.

Mit der Einrichtung 18 kann nicht nur ein Konzentratbolus auf der Dialysierflüssigkeitsseite, sondern auch ein Temperaturbolus auf der Blutseite erzeugt werden. Hierzu erhöht die Einrichtung 18 kurzzeitig die Temperatur des in die Dialysierflüssigkeitskammer strömenden Blutes, wodurch kurzzeitig die Temperatur des in Gegenrichtung aus der Blutkammer strömenden Blutes ebenfalls erhöht wird.

Nachfolgend wird die Funktion der Vorrichtung und das Prinzip der Messung im Einzelnen erläutert.

Das vom linken Ventrikel 1 emittierte Blut fließt zum größten Teil in die Kapillarsysteme aller Organe, zu einem kleinen Teil in die Fistel 5. Für den Fall, dass der Blutfluss im extrakorporalen Kreislauf 7 kleiner als der Blutfluss des in die Fistel bzw. aus der Fistel fließenden Blutes ist, fließt das Fistelblut zu einem Teil durch den extrakorporalen Kreislauf 7, zum anderen Teil durch die Fistel 5.

Wenn der extrakorporale Blutfluss größer als der Fistelfluss ist, dann rezirkuliert Blut aus dem extrakorporalen Kreislauf, wobei die Fistel vom venösen zum arteriellen Anschluss durchflossen wird. Das venöse Blut, das durch die Fistel fließende Blut und das aus den Kapillarsystemen stammende Blut vereinigt sich schließlich wieder im Rücklauf zum Herzen.

Die erste Einrichtung zur Überwachung der Rezirkulation 27, die mit der Steuereinheit 23 in Verbindung steht, leitet die Messung dadurch ein, dass die Einrichtung 18 einen Temperaturbolus erzeugt. Der Dialysator 8, dessen Dialysierflüssigkeitsrate höher als die Blutflussrate ist, und dessen Dialysierflüssigkeit in Gegenrichtung zum Blut fließt, überträgt den Temperaturbolus $T_{Dia}$ auf das Blut am Auslass der Blutkammer 10. Der Temperaturbolus $T_{ven}$ im venösen Zweig 20 des extrakorporalen Blutkreislaufs 7 wird mit der venösen Messeinrichtung 26 gemessen. Der Temperaturbolus breitet sich dann entlang der zwei Rezirkulationswege aus und wird an den Stellen, an denen sich der Fluss vermischt, in seiner Höhe reduziert. Ein kleinerer Antwortbolus $T_{art}$ wird dann mit der arteriellen Messeinrichtung 25 gemessen. Die erste Überwachungseinrichtung 27 berechnet das Verhältnis des gemessenen arteriellen und venösen Temperaturbolus. Dieses Verhältnis ist gleich der totalen Rezirkulation, d.h. der Summe aus fistel- und kardiopulmonärer Rezirkulation.

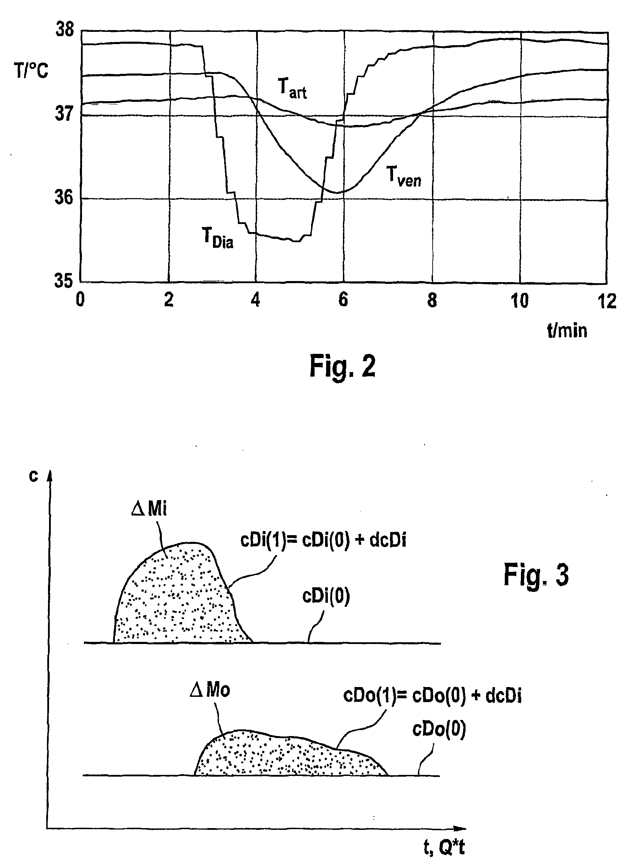

Figur 2 zeigt die oben beschriebenen Temperaturverläufe. Zur Erzeugung des Bolus wird die Dialysattemperatur für 2,5 min um 2,5° gesenkt. Mit einer Verzögerung wird die venöse Bluttemperatur $T_{ven}$ davon beeinflusst. Nach einer weiteren kurzen Verzögerung wird der arterielle Antwortbolus $T_{art}$ beobachtet. In dem Beispiel von Figur 2 ergibt sich aus dem Verhältnis der Bolushöhe eine totale Rezirkulaton von 22 %.

Die zweite Einrichtung 29 zur Überwachung der Rezirkulation leitet zu Beginn der Messung mit der Einrichtung 18 eine kurzzeitige Erhöhung der Ionenkonzentration der in die Dialysierflüssigkeitskammer 9 des Dialysators 8 strömenden Dialysierflüssigkeit ein, die mit der Messeinrichtung 13 gemessen wird. Die auf den Konzentratbolus zurückzuführende Veränderung der Ionenkonzentration der aus der Dialysierflüssigkeitskammer strömenden Dialysierflüssigkeit wird mit der Messeinrichtung 17 gemessen.

Figur 3 zeigt den zeitlichen Verlauf der Veränderung der Dialysierflüssigkeitseingangs- und -ausgangskonzentration $C_{di}$ und $C_{do}$. Deutlich ist zu erkennen, dass der Konzentratbolus am Eingang des Dialysators mit einer zeitlichen Verzögerung am Ausgang des Dialysators auftritt. Die Amplitude des Konzentratbolus am Ausgang ist geringer als am Eingang des Dialysators. Dabei ist nur der Teil des Konzentratbolus zu berücksichtigen, der nicht einer Basis- oder Grundkonzentration zuzuschreiben ist. Zur Ermittlung der Basiskonzentration können Messwerte vor und/oder nach dem Bolus herangezogen werden.

Die zweite Überwachungseinrichtung 29 verfügt über eine Recheneinheit, die aus dem zeitlichen Verlauf der Dialysierflüssigkeitsionenkonzentration stromauf und stromab des Dialysators und der durch die Steuereinheit 23 vorgegebenen Dialysierflüssigkeitsrate $Q_d$, die der Flussrate der Dialysierflüssigkeitspumpe 15 entspricht, die beiden Größen $\Delta M_i$ und $\Delta M_o$ nach den folgenden Gleichungen berechnet:

$$\Delta Mi = Qd * \int dcdi * dt \qquad (1)$$

$$\Delta Mo = Qd * \int dcdo * dt \qquad (2).$$

Die Dialysance D wird aus den Größen $\Delta M_i$ und $\Delta M_o$ nach der folgenden Gleichung berechnet:

$$D = QD * \frac{\Delta Mi - \Delta Mo}{\Delta Mi} \qquad (3).$$

Während der Dialysebehandlung führt die zweite Überwachungseinrichtung 29 fortlaufend zu bestimmten Zeitpunkten Messungen zur Bestimmung der Dialysance durch. Die Werte der Dialysance werden in der Auswerteinrichtung 32 ausgewertet.

Die Auswerteinrichtung 32 verfügt über eine Speichereinheit zum Speichern eines vorgegebenen Wertes der Dialysance, beispielsweise einen für eine effektive Dialysebehandlung spezifischen Dialysancewert und eine Vergleichseinheit zum Vergleichen des ermittelten Dialysancewertes mit dem vorgegebenen Dialysancewert. Wenn die Differenz zwischen dem vorgegebenen Wert und dem ermittelten Wert größer als ein vorgegebener Grenzwert ist, beispielsweise eine prozentuale Abweichung von mehr als 10 bis 30 %, vorzugsweise 15 %, wird die erste Überwachungseinrichtung 27 automatisch aktiviert.

Wenn sich die Dialysance um mehr als den vorgegebenen Betrag verringert hat, führt die erste Überwachungseinrichtung 27 eine Messung der Rezirkulation durch. Der ermittelte Wert der Rezirkulation wird mit einem vorgegebenen Rezirkulationswert verglichen, der ebenfalls in der Speichereinheit gespeichert ist. Die vorgegebenen Dialyse- und Rezirkulationswerte können auch patientenspezifisch auf einer Patientenkarte abgelegt sein.

Wenn der Betrag der Differenz zwischen dem vorgegebenen Rezirkulationswert und dem ermittelten Rezirkulationswert größer als ein vorgegebener Grenzwert ist, beispielsweise sich die Rezirkulation um mehr als 10 bis 30 %, vorzugsweise 20 % vergrößert hat, wird ein erstes Steuersignal erzeugt, wenn die Differenz kleiner als der vorgegebene Grenzwert ist, beispielsweise sich die Rezirkulation nicht verändert oder nur geringfügig verändert hat, wird ein zweites Steuersignal erzeugt. Die beiden Steuersignale werden von der Steuereinheit 23 empfangen, um entsprechende Maßnahmen einzuleiten. Es kann vorgesehen sein, dass ein Eingriff in die Maschinensteuerung vorgenommen wird. Darüber hinaus kann die Alarm- oder Anzeigeeinrichtung 37 angesteuert werden, die signalisiert, dass entweder eine Komplikation aufgetreten ist, die auf den Dialysator oder auf den Gefäßzugang zurückzuführen ist.

Ein fehlerhafter Gefäßzugang wird dann angenommen, wenn mit der ersten Überwachungseinrichtung 29 eine signifikante Reduzierung der Dialysance oder Clearance und mit der zweiten Überwachungseinrichtung 27 eine signifikante Verringerung der Rezirkulation festgestellt wird.

## Patentansprüche

1. Vorrichtung zur Erkennung von Komplikationen während einer extrakorporalen Blutbehandlung mit einer Blutbehandlungsvorrichtung, bei der das zu behandelnde Blut in einem extrakorporalen Blutkreislauf die Blutkammer eines durch eine semipermeable Membran in die Blutkammer und eine Dialysierflüssigkeitskammer unterteilten Dialysators durchströmt und Dialysierflüssigkeit in einem Dialysierflüssigkeitskreislauf die Dialysierflüssigkeitskammer des Dialysators durchströmt, wobei die Überwachungsvorrichtung aufweist:

   Mittel (18) zum Verändern einer physikalischen oder chemischen Kenngröße des Blutes im extrakorporalen Blutkreislauf (6),
   Mittel (18) zum Verändern einer physikalischen oder chemischen Kenngröße der Dialysierflüssigkeit im Dialysierflüssigkeitskreislauf (7),
   Mittel (13, 17; 25, 26) zum Messen von physikalischen oder chemischen Kenngrößen des Blutes oder der Dialysierflüssigkeit,
   eine erste Einrichtung (27) zur Überwachung der Rezirkulation der Blutbehandlung, die derart mit den Mitteln zum Verändern einer physikalischen oder chemischen Kenngröße des Blutes und den Mitteln zum Messen einer physikalischen oder chemischen Kenngröße des Blutes oder der Dialysierflüssigkeit zusammenwirkt, dass die Rezirkulation auf der Grundlage einer Veränderung der gemessenen physikalischen oder chemischen Kenngröße des Blutes überwachbar ist,
   eine Einrichtung (29) zur Überwachung der Dialysance oder Clearance der Blutbehandlung, die derart mit den Mitteln zum Verändern einer physikalischen oder chemischen Kenngröße der Dialysierflüssigkeit und den Mitteln zum Messen einer physikalischen oder chemischen Kenngröße des Blutes oder der Dialysierflüssigkeit zusammenwirkt, dass die Dialysance oder Clearance auf der Grundlage einer Veränderung der gemessenen physikalischen oder chemischen Kenngröße der Dialysierflüssigkeit überwachbar ist,
   **gekennzeichnet durch**,
   dass die Einrichtung (29) zur Überwachung der Dialysance oder Clearance als zweite Einrichtung zur Überwachung der Rezirkulation ausgebildet ist, wobei die Rezirkulation auf der Grundlage der Feststellung der Veränderung der Dialysance oder Clearance überwachbar ist und
eine Auswerteinrichtung (32), die derart mit der ersten und zweiten Einrichtung (27, 29) zur Bestimmung der Rezirkulation zusammenwirkt, dass während der Blutbehandlung die zweite Einrichtung (29) zur Überwachung der Rezirkulation zu aufeinanderfolgenden Zeitpunkten aktivierbar ist, wobei nach der Feststellung der Veränderung der Rezirkulation mit der zweiten Einrichtung die erste Einrichtung aktivierbar ist, so dass das Auftreten einer Komplikation detektierbar ist.

2. Vorrichtung nach Anspruch 1, **dadurch gekennzeichnet, dass** die Auswerteinrichtung (32) Mittel zum Erzeugen eines Steuersignals bei dem Auftreten einer Komplikation aufweist.

3. Vorrichtung nach Anspruch 2, **dadurch gekennzeichnet, dass** die Mittel zum Erzeugen eines Steuersignals derart ausgebildet sind, dass ein erstes Steuersignal erzeugt wird, wenn nach der Feststellung einer Veränderung der Rezirkulation mit der zweiten Überwachungseinrichtung (29) die erste Überwachungseinrichtung (27) eine Veränderung der Rezirkulation feststellt.

4. Vorrichtung nach Anspruch 3, **dadurch gekennzeichnet, dass** beim Erzeugen des ersten Steuersignals Mittel zum Erzeugen eines Alarms und/oder zum Vornehmen eines Eingriffs in die Maschinensteuerung der Blutbehandlungsvorrichtung aktivierbar sind.

5. Vorrichtung nach einem der Ansprüche 2 bis 4, **dadurch gekennzeichnet, dass** die Mittel zum Erzeugen eines Steuersignals derart ausgebildet sind, dass ein zweites Steuersignal erzeugt wird, wenn nach der Feststellung einer Veränderung der Rezirkulation mit der zweiten Überwachungseinrichtung (29) die erste Überwachungseinrichtung (27) eine Veränderung der Rezirkulation nicht feststellt.

6. Vorrichtung nach Anspruch 5, **dadurch gekennzeichnet, dass** beim Erzeugen des zweiten Steuersignals Mittel zum Erzeugen eines Alarms und/oder zum Vornehmen eines Eingriffs in die Maschinensteuerung der Blutbehandlungsvorrichtung aktivierbar sind.

7. Vorrichtung nach einem der Ansprüche 1 bis 6, **dadurch gekennzeichnet, dass** die Auswerteinrichtung (32) eine Speichereinheit zum Speichern eines vorgegebenen Wertes der Dialysance oder Clearance oder Rezirkulation und eine Vergleichseinheit zum Vergleichen des zu einem bestimmten Zeitpunkt ermittelten Dialysance-, Clearance- oder Rezirkulationswertes mit dem vorgegebenen Wert aufweist, wobei eine Veränderung der Rezirkulation, Dialysance der Clearance feststellbar ist, wenn der Betrag der Differenz zwischen dem vorgegebenen und zu einem bestimmten Zeitpunkt ermittelten Wert größer als ein vorgegebener Grenzwert ist.

8. Vorrichtung nach einem der Ansprüche 1 bis 7, **dadurch gekennzeichnet, dass** die erste Einrichtung (27) zur Überwachung der Rezirkulation Mittel (18) zum Verändern der Temperatur des Blutes und Mittel (25, 26) zum Messen der Temperatur des Blutes im Blutkreislauf aufweist.

9. Vorrichtung nach einem der Ansprüche 1 bis 8, **dadurch gekennzeichnet, dass** die zweite Einrichtung (29) zur Überwachung der Rezirkulation Mittel (18) zum Verändern der Konzentration einer Substanz in der Dialysierflüssigkeit und Mittel (13, 17) zum Messen der Konzentration der Substanz in der Dialysierflüssigkeit im Dialysierflüssigkeitskreislauf aufweist.

10. Blutbehandlungsvorrichtung mit
einem durch eine semipermeable Membran (8a) in eine Blutkammer (10) und eine Dialysierflüssigkeitskammer (9) unterteilten Dialysator (8), wobei die Blutkammer (10) in einen extrakorporalen Blutkreislauf (7) und die Dialysierflüssigkeitskammer (9) des Dialysators (8) in einen Dialysierflüssigkeitskreislauf (6) geschaltet sind und
einer Vorrichtung zur Erkennung von Komplikationen nach einem der Ansprüche 1 bis 9.

11. Verfahren zur Erkennung von Komplikationen während einer extrakorporalen Blutbehandlung, bei der das zu behandelnde Blut in einem extrakorporalen Blutkreislauf die Blutkammer eines durch eine semipermeable Membran in die Blutkammer und eine Dialysierflüssigkeitskammer unterteilten Dialysators durchströmt und Dialysierflüssigkeit in einem Dialysierflüssigkeitskreislauf die Dialysierflüssigkeitskammer des Dialysators durchströmt,
**dadurch gekennzeichnet,**
**dass** während der Blutbehandlung eine physikalische oder chemische Kenngröße der Dialysierflüssigkeit im Dialysierflüssigkeitskreislauf verändert und eine physikalische oder chemische Kenngröße der Dialysierflüssigkeit gemessen wird, wobei zu aufeinanderfolgenden Zeitpunkten die Dialysance oder Clearance auf der Grundlage der Veränderung der gemessenen physikalischen oder chemischen Kenngröße der Dialysierflüssigkeit im Dialysierflüssigkeitskreislauf überwacht wird, und bei einer Veränderung der Dialysance oder Clearance zur Feststellung einer Komplikation während der Blutbehandlung eine physikalische oder chemische Kenngröße des Blutes im extrakorporalen Blutkreislauf verändert und eine physikalische oder chemische Kenngröße des Blutes gemessen wird, wobei die Rezirkulation auf der Grundlage der Veränderung der gemessenen physikalischen oder chemischen Kenngröße des Blutes im Blutkreislauf überwacht wird.

12. Verfahren nach Anspruch 11, **dadurch gekennzeichnet, dass** bei dem Auftreten einer Komplikation ein Steuersignal erzeugt wird.

13. Verfahren nach Anspruch 12, **dadurch gekennzeichnet, dass** ein erstes Steuersignal erzeugt wird, wenn nach der Feststellung einer Veränderung der Rezirkulation auf der Grundlage einer Veränderung der gemessenen Kenngröße der Dialysierflüssigkeit eine Veränderung der Rezirkulation auf der Grundlage der gemessenen Veränderung der Kenngröße des Blutes festgestellt wird.

14. Verfahren nach Anspruch 13, **dadurch gekennzeichnet, dass** beim Erzeugen des ersten Steuersignals ein Alarm gegeben und/oder ein Eingriff in die Maschinensteuerung der Blutbehandlungsvorrichtung vorgenommen wird.

15. Verfahren nach einem der Ansprüche 12 bis 14, **dadurch gekennzeichnet, dass** ein zweites Steuersignal erzeugt wird, wenn nach der Feststellung einer Veränderung der Rezirkulation auf der Grundlage der Veränderung der gemessenen Kenngröße der Dialysierflüssigkeit eine Rezirkulation auf der Grundlage einer Veränderung einer gemessenen Kenngröße des Blutes nicht festgestellt wird.

16. Verfahren nach Anspruch 15, **dadurch gekennzeichnet, dass** beim Erzeugen des zweiten Steuersignals ein Alarm gegeben und/oder ein Eingriff in die Maschinensteuerung der Blutbehandlungsvorrichtung vorgenommen wird.

17. Verfahren nach einem der Ansprüche 11 bis 16, **dadurch gekennzeichnet, dass** zur Überwachung der Rezirkulaton oder Dialysance oder Clearance ein vorgegebener Wert der Rezirkulation, Dialysance oder Clearance gespeichert und der zu einem bestimmten Zeitpunkt ermittelte Rezirkulations-, Dialysance- oder Clearancewert mit dem vorgegebenen Wert verglichen wird, wobei eine Veränderung der Rezirkulation, Dialysance oder Clearance festgestellt wird, wenn die Differenz zwischen dem vorgegebenen und zu einem bestimmten Zeitpunkt ermittelten Wert größer als ein vorgegebener Grenzwert ist.

18. Verfahren nach einem der Ansprüche 11 bis 17, **dadurch gekennzeichnet, dass** zur Überwachung der Rezirkulation die Temperatur des Blutes verändert und die Temperatur des Blutes im Blutkreislauf gemessen wird.

19. Verfahren nach einem der Ansprüche 11 bis 18, **dadurch gekennzeichnet, dass** zur Überwachung der Dialysance oder Clearance die Konzentration einer Substanz in der Dialysierflüssigkeit verändert und die Konzentration der Substanz im Dialysierflüssigkeitskreislauf gemessen wird.

**Claims**

1. Device for detecting complications during an extracorporeal blood treatment using a blood treatment device, in which the blood to be treated flows in an extracorporeal blood circuit through the blood chamber of a dialyzer which is subdivided by a semipermeable membrane into the blood chamber and a dialysis fluid chamber, and dialysis fluid flows in a dialysis fluid circuit through the dialysis fluid chamber of the dialyzer, the monitoring device having:

means (18) for changing a physical or chemical parameter of the blood in the extracorporeal blood circuit (6),
means (18) for changing a physical or chemical parameter of the dialysis fluid in the dialysis fluid circuit (7),
means (13, 17; 25, 26) for measuring physical or chemical parameters of the blood or of the dialysis fluid,
a first unit (27) for monitoring the recirculation of the blood treatment, which cooperates with the means for changing a physical or chemical parameter of the blood and the means for measuring a physical or chemical parameter of the blood or of the dialysis fluid in such a way that the recirculation is monitorable based on a change in the measured physical or chemical parameter of the blood,
a unit (29) for monitoring the dialysance or clearance of the blood treatment, which cooperates with the means for changing a physical or chemical parameter of the dialysis fluid and the means for measuring a physical or chemical parameter of the blood or of the dialysis fluid in such a way that the dialysance or clearance is monitorable based on a change in the measured physical or chemical parameter of the dialysis fluid,
**characterized in that**
the unit (29) for monitoring the dialysance or clearance is designed as a second unit for monitoring the recirculation, the recirculation being monitorable based on the ascertainment of the change in the dialysance or clearance, and
an evaluation device (32) which cooperates with the first and second units (27, 29) for determining the recirculation in such a way that during the blood treatment the second unit (29) for monitoring the recirculation is activatable at successive points in time, whereby after the change in the recirculation is ascertained using the second unit, the first unit is activatable so that the occurrence of a complication is detectable.

2. Device according to Claim 1, **characterized in that** the evaluation device (32) has means for generating a control signal when a complication occurs.

3. Device according to Claim 2, **characterized in that** the means for generating a control signal are designed in such a way that a first control signal is generated when, after a change in the recirculation is ascertained using the second monitoring device (29), the first monitoring device (27) ascertains a change in the recirculation.

4. Device according to Claim 3, **characterized in that** when the first control signal is generated, means are activatable for generating an alarm and/or for making an intervention in the machine control of the blood treatment device.

5. Device according to one of Claims 2 through 4, **characterized in that** the means for generating a control signal are designed in such a way that a second control signal is generated when, after a change in the recirculation is ascertained using the second monitoring device (29), the first monitoring device (27) does not ascertain a change in the recirculation.

6. Device according to Claim 5, **characterized in that** when the second control signal is generated, means are activatable for generating an alarm and/or for making an intervention in the machine control of the blood treatment device.

7. Device according to one of Claims 1 through 6, **characterized in that** the evaluation device (32) has a memory unit for storing a predefined value of the dialysance or clearance or recirculation, and has a comparator for comparing the dialysance, clearance, or recirculation value which is determined at a certain point in time to the predefined value, a change in the recirculation, dialysance, or clearance being ascertainable when the absolute value of the difference between the predefined value and the value which is determined at a certain point in time is greater than a predefined limit value.

8. Device according to one of Claims 1 through 7, **characterized in that** the first unit (27) for monitoring the recirculation has means (18) for changing the temperature of the blood, and has means (25, 26) for measuring the temperature of the blood in the blood circuit.

9. Device according to one of Claims 1 through 8, **characterized in that** the second unit (29) for monitoring the recirculation has means (18) for changing the concentration of a substance in the dialysis fluid, and has means (13, 17) for measuring the concentration of the substance in the dialysis fluid in the dialysis fluid circuit.

10. Blood treatment device, having
a dialyzer (8) which is subdivided by a semipermeable membrane (8a) into a blood chamber (10) and a dialysis fluid chamber (9), the blood chamber (10) being connected to an extracorporeal blood circuit (7), and the dialysis fluid chamber (9) of the dialyzer (8) being connected to a dialysis fluid circuit (6), and
a device for detecting complications according to one of Claims 1 through 9.

11. Method for detecting complications during an extracorporeal blood treatment, in which the blood to be treated flows in an extracorporeal blood circuit through the blood chamber of a dialyzer which is subdivided by a semipermeable membrane into the blood chamber and a dialysis fluid chamber, and dialysis fluid flows in a dialysis fluid circuit through the dialysis fluid chamber of the dialyzer,
**characterized in that**
during the blood treatment, a physical or chemical parameter of the dialysis fluid in the dialysis fluid circuit is changed and a physical or chemical parameter of the dialysis fluid is measured, the dialysance or clearance being monitored at successive points in time based on the change in the measured physical or chemical parameter of the dialysis fluid in the dialysis fluid circuit, and when there is a change in the dialysance or clearance, for ascertaining a complication during the blood treatment a physical or chemical parameter of the blood in the extracorporeal blood circuit is changed and a physical or chemical parameter of the blood is measured, the recirculation being monitored based on the change in the measured physical or chemical parameter of the blood in the blood circuit.

12. Method according to Claim 11, **characterized in that** a control signal is generated when a complication occurs.

13. Method according to Claim 12, **characterized in that** a first control signal is generated when, after a change in the recirculation is ascertained based on a change in the measured parameter of the dialysis fluid, a change in the recirculation based on the measured change in the parameter of the blood is ascertained.

14. Method according to Claim 13, **characterized in that** when the first control signal is generated, an alarm is provided and/or an intervention is made in the machine control of the blood treatment device.

15. Method according to one of Claims 12 through 14, **characterized in that** a second control signal is generated when, after a change in the recirculation is ascertained based on the change in the measured parameter of the dialysis fluid, a recirculation based on a change in a measured parameter of the blood is not ascertained.

16. Method according to Claim 15, **characterized in that** when the second control signal is generated, an alarm is provided and/or an intervention is made in the machine control of the blood treatment device.

17. Method according to one of Claims 11 through 16, **characterized in that** for monitoring the recirculation or dialysance or clearance, a predefined value of the recirculation, dialysance, or clearance is stored, and the recirculation, dialysance, or clearance value determined at a certain point in time is compared to the predefined value, a change in the recirculation, dialysance, or clearance being ascertained when the difference between the predefined value and the value determined at a certain point in time is greater than a predefined limit value.

18. Method according to one of Claims 11 through 17, **characterized in that** for monitoring the recirculation, the temperature of the blood is changed, and the temperature of the blood in the blood circuit is measured.

19. Method according to one of Claims 11 through 18, **characterized in that** for monitoring the dialysance or clearance, the concentration of a substance in the dialysis fluid is changed, and the concentration of the substance in the dialysis fluid circuit is measured.

**Revendications**

1. Dispositif pour l'identification de complications pendant un traitement sanguin extracorporel avec un dispositif de traitement sanguin dans lequel le sang à traiter, dans un circuit sanguin extracorporel, traverse la chambre de sang d'un dialyseur subdivisé par une membrane semi-perméable en la chambre de sang et en une chambre de liquide de dialyse et un liquide de dialyse, dans un circuit de liquide de dialyse, traverse la chambre de liquide de dialyse du dialyseur, dans lequel le dispositif de surveillance présente :

des moyens (18) pour modifier une caractéristique physique ou chimique du sang dans le circuit sanguin extracorporel (6) :

des moyens (18) pour modifier une caractéristique physique ou chimique du liquide de dialyse dans le circuit du liquide de dialyse (7),
des moyens (13, 17 ; 25, 26) pour mesurer des caractéristiques physiques ou chimiques du sang ou du liquide de dialyse,
un premier appareil (27) de surveillance de la recirculation du traitement sanguin qui coopère de telle sorte avec les moyens de modification d'une caractéristique physique ou chimique du sang et les moyens de mesure d'une caractéristique physique ou chimique du sang ou du liquide de dialyse que la recirculation peut être surveillée sur la base d'une modification de la caractéristique physique ou chimique mesurée du sang un appareil (29) de surveillance de la dialysance ou de la clairance du traitement sanguin qui coopère de telle sorte avec les moyens de modification d'une caractéristique physique ou chimique du liquide de dialyse et les moyens de mesure d'une caractéristique physique ou chimique du sang ou du liquide de dialyse que la dialysance ou la clairance peut être surveillée sur la base d'une modification de la caractéristique physique ou chimique mesurée du liquide de dialyse,

**caractérisé en ce que**
l'appareil (29) de surveillance de la dialysance ou de la clairance est conçu comme un deuxième appareil de surveillance de la recirculation, dans lequel la recirculation peut être surveillée sur la base de la constatation de la modification de la dialysance ou de la clairance et

un appareil d'évaluation (32) qui coopère de telle sorte avec le premier et le deuxième appareil (27, 29) pour la détermination de la recirculation, que pendant le traitement sanguin, le deuxième appareil (29) peut être activé pour la surveillance de la recirculation à des moments consécutifs, dans lequel après la détermination de la modification de la recirculation avec le deuxième appareil, le premier appareil peut être activé de telle sorte que la survenue d'une complication peut être détectée.

2.   Dispositif selon la revendication 1, **caractérisé en ce que** l'appareil d'évaluation (32) présente des moyens de génération d'un signal de commande lors de la survenue d'une complication.

3.   Dispositif selon la revendication 2, **caractérisé en ce que** les moyens de génération d'un signal de commande sont conçus de telle sorte qu'un premier signal de commande est généré lorsque, après la constatation d'une modification de recirculation avec le deuxième appareil de surveillance (29), le premier appareil de surveillance (27) constate une modification de la recirculation.

4.   Dispositif selon la revendication 3, **caractérisé en ce que**, lors de la génération du premier signal de commande, des moyens de génération d'une alarme et/ou d'engagement d'une intervention peuvent être activés dans la commande de la machine du dispositif de traitement sanguin.

5.   Dispositif selon l'une des revendications 2 à 4, **caractérisé en ce que** les moyens de génération d'un signal de commande sont conçus de tels sorte qu'un deuxième signal de commande est généré, lorsque, après la constatation d'une modification de la recirculation avec le deuxième appareil de surveillance (29), le premier dispositif de surveillance (27) ne constate pas de modification de la recirculation.

6.   Dispositif selon la revendication 5, **caractérisé en ce que**, lors de la génération du deuxième signal de commande, des moyens de génération d'une alarme et/ou d'engagement d'une intervention peuvent être activés dans la commande de la machine du dispositif de traitement sanguin.

7.   Dispositif selon l'une des revendications 1 à 6, **caractérisé en ce que** l'appareil d'évaluation (32) présente une unité de mémoire pour mémoriser une valeur de dialysance ou de clairance ou de recirculation prédéterminée, et une unité de comparaison pour la comparaison de la valeur de dialysance, de clairance ou de recirculation déterminée à un moment donné à la valeur prédéterminée, dans lequel une modification de la recirculation, de la dialysance, de la clairance peut être constatée lorsque le niveau de différence entre la valeur prédéterminée et la valeur déterminée à un moment donné est supérieur à une valeur seuil prédéterminée.

8.   Dispositif selon l'une des revendications 1 à 7, **caractérisé en ce que** le premier appareil (27) de surveillance de la recirculation présente des moyens (18) de modification de la température du sang et des moyens (25, 26) de mesure de la température du sang dans la circulation sanguine.

9.   Dispositif selon l'une des revendications 1 à 8, **caractérisé en ce que** le deuxième appareil (29) présente, pour la surveillance de la recirculation, un moyen (18) de modification de la concentration d'une substance dans le liquide de dialyse et des moyens (13, 17) de mesure de la concentration de la substance dans le liquide de dialyse dans la circulation de liquide de dialyse.

10.   Dispositif de traitement sanguin comportant
un dialyseur (8) subdivisé par une membrane semi-perméable (8a) en une chambre de sang (10) et une chambre de liquide de dialyse (9), dans lequel la chambre de sang (10) dans une circulation sanguine extracorporelle (7) et la chambre de liquide de dialyse (9) du dialyseur (8) dans une circulation de liquide de dialyse (6) sont commutées et un dispositif d'identification de complications selon l'une des revendications 1 à 9.

11.   Procédé d'identification de complications pendant un traitement sanguin extracorporel, lors duquel le sang à traiter dans une circulation sanguine extracorporelle traverse la chambre de sang d'un dialyseur subdivisé par une membrane semi-perméable en la chambre de sang et en une chambre de liquide de dialyse et un liquide de dialyse, dans un circuit de liquide de dialyse, traverse la chambre de liquide de dialyse du dialyseur, **caractérisé en ce que**, pendant le traitement sanguin, une caractéristique physique ou chimique du liquide de dialyse dans la circulation de liquide de dialyse est modifiée et une caractéristique physique ou chimique du liquide de dialyse est mesurée, dans lequel, à des moments consécutifs, la dialysance ou la clairance est surveillée sur la base de la modification de la caractéristique physique ou chimique mesurée du liquide de dialyse dans la circulation du liquide de dialyse, et lors d'une modification de la dialysance ou de la clairance pour la constatation d'une complication pendant le traitement sanguin, une caractéristique physique ou chimique du sang dans la circulation extracorporelle est modifiée et une caractéristique physique ou chimique du sang est mesurée, dans lequel la recirculation est surveillée sur la base de la modification de la caractéristique physique ou chimique mesurée du sang dans la circulation sanguine.

**12.** Procédé selon la revendication 11, **caractérisé en ce que**, lors de la survenue d'une complication, un signal de commande est généré.

**13.** Procédé selon la revendication 12, **caractérisé en ce qu'**un premier signal de commande est généré lorsque, après la constatation d'une modification de recirculation sur la base d'une modification de la caractéristique mesurée du liquide de dialyse, une modification de recirculation est constatée sur la base de la modification mesurée de la caractéristique du sang.

**14.** Procédé selon la revendication 13, **caractérisé en ce que**, lors de la génération du premier signal de commande, une alarme est émise et/ou une intervention dans la commande de la machine du dispositif de traitement sanguin est engagée.

**15.** Procédé selon l'une des revendications 12 à 14, **caractérisé en ce qu'**un deuxième signal de commande est généré, lorsque, après la constatation d'une modification de recirculation sur la base de la modification de la caractéristique mesurée du liquide de dialyse, une recirculation n'est pas constatée sur la base d'une modification d'une caractéristique du sang mesurée.

**16.** Procédé selon la revendication 15, **caractérisé en ce que**, lors de la génération du deuxième signal de commande, une alarme est émise et/ou une intervention dans la commande de la machine du dispositif de traitement sanguin est engagée.

**17.** Procédé selon l'une des revendications 11 à 16, **caractérisé en ce que**, pour la surveillance de la recirculation ou de la dialysance ou de la clairance, une valeur de recirculation, de dialysance ou de clairance prédéterminée est mémorisée et la valeur de recirculation, de dialysance ou de clairance déterminée à un moment donné est comparée à la valeur prédéterminée, dans lequel une modification de la recirculation, de la dialysance ou de la clairance est constatée lorsque la différence entre la valeur prédéterminée et la valeur déterminée à un moment donné est supérieure à une valeur seuil prédéterminée.

**18.** Procédé selon l'une des revendications 11 à 17, **caractérisé en ce que**, pour la surveillance de la recirculation, la température du sang est modifiée et la température du sang dans la circulation sanguine est mesurée.

**19.** Procédé selon l'une des revendications 11 à 18, **caractérisé en ce que**, pour la surveillance de la dialysance ou de la clairance, la concentration d'une substance dans le liquide de dialyse est modifiée et la concentration de la substance dans la circulation du liquide de dialyse est mesurée.

Fig. 1

**Fig. 2**

**Fig. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19702441 C1
- DE 19702441
- US 6702774 B1
- DE 19747360 A1
- DE 3938662

**In der Beschreibung aufgeführte Nicht-Patentliteratur**

- Models to predict recirculation and its effect on treatment time in single-needle-dialysis. **F. GOTCH.** First Intl. Symposium on Single-Needle-Dialyse. ISAO Press, 1984, 305 ff
- **KRÄMER ; POLASCHEGG.** *Zeitschrift EDTNA-ERCA Journal,* April 1993, vol. XIX (2), 8-15